# NOUVEAU FASCICULE DE BREVET EUROPEEN

(11) **EP 0 938 887 B2**
(45) Date de publication et mention de la décision concernant l'opposition: **13.02.2008**
(45) Mention de la délivrance du brevet: 19.11.2003
(21) Numéro de dépôt: 98402849.8
(22) Date de dépôt: 17.11.1998
(51) Int. Cl.: A61K 8/49, A61Q 5/00, A61Q 19/00

(54) **Utilisation d'agent de coloration photochrome dans une composition cosmétique, et composition cosmétique le comprenant**
Verwendung von einem photochromischem Farbstoff in einer kosmetischen Zusammensetzung und kosmetische Zusammensetzung enthaltend diesen Farbstoff
Use of a photochromic colouring agent in a cosmetic composition and cosmetic composition containing it

(30) Priorité: 12.12.1997 FR 9715769
(43) Date de publication de la demande: 01.09.1999
(73) Titulaire: L'ORÉAL, 75008 Paris (FR)
(72) Inventeur: Lagrange, Alain, 77700 Coupvray (FR)
(74) Mandataire: Dossmann, Gérard

(56) Documents cités:
- EP-A- 0 336 193
- EP-A- 0 359 909
- EP-A- 0 562 201
- FR-A- 1 604 929
- FR-A- 2 088 003
- JP-A- 3 261 762
- JP-A- 56 100 709
- JP-A- 61 275 209
- US-A- 5 208 132
- BASE DE DONN¹ES "CHEMICAL ABSTRACTS" (SERVEUR: STN),Abr.:105: 208 778, Colombus, OH, USA; & JP 61 076 490 (SHISEIDO CO., LTD) 18 AVRIL 1986 XP002072882
- Photochromie CD Römpp Chemie Lexikon-Version 1.0,1995

## Description

La présente invention concerne une nouvelle composition cosmétique, en particulier une composition cosmétique de maquillage ou une composition capillaire, comprenant un agent de coloration particulier ayant des propriétés photochromes, et permettant d'obtenir des effets de coloration nouveaux.

Les compositions de maquillage telles que les poudres libres ou compactes, les fonds de teint, les fards à joues ou à paupières, les rouges à lèvres ou les vernis à ongles, sont constituées d'un véhicule approprié et de différents agents de coloration destinés à conférer une certaine couleur auxdites compositions avant et/ou après leur application sur la peau, les muqueuses, les semi-muqueuses et/ou les phanères telles que les ongles ou les cheveux.
Pour créer des couleurs, on utilise aujourd'hui une gamme d'agents de coloration assez limitée et notamment des laques, des pigments minéraux ou des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles. Pour obtenir des effets colorés, on peut encore employer des pigments nacrés de couleurs variées, mais jamais intenses, qui permettent d'obtenir des effets irisés mais le plus souvent assez faibles.

Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments usuels pour apporter un reflet temporaire aux cheveux, mais les nuances obtenues par cette coloration restent assez ternes, trop uniformes et peu ludiques. On peut notamment assimiler une telle coloration à un "maquillage" des cheveux.

Il a alors été proposé d'utiliser des composés photochromes dans les compositions de maquillage ou capillaire, de manière à obtenir des changements agréables et variables dans le 'rendu couleur' des maquillages de la peau et/ou des cheveux.
Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint et les fards à joues ou à paupières. En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'opposé, un maquillage réalisé à l'extérieur paraîtra plus sombre dans un endroit éclairé artificiellement.
Pour pallier ce problème, il a été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.
Il a également été proposé d'utiliser des composés photochromes organiques, tels que les composés de la famille des spiropyrannes ou des naphtooxazines.
Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse.
On peut ainsi citer le brevet FR1604929 qui décrit des compositions cosmétiques notamment capillaires sous forme d'aérosol, qui contiennent des composés phototropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.
Les compositions comprenant ces composés ont toutefois la faculté de changer constamment de couleur en fonction de l'intensité lumineuse.

Le document brevet FR-A-2 088 003 décrit une composition cosmétique contenant un agent de coloration photochrome de type fulgide. Cependant, ce document décrit de façon générale les fulgides, sans distinction entre les fulgides thermiquement réversibles ou irréversibles.

Or, il subsiste le besoin de disposer d'agents de coloration qui permettent d'une part d'obtenir une coloration qui ne change pas, ou peu, que l'on soit en présence ou en l'absence d'UV, comme le feraient des pigments usuels, tout en conservant la possibilité de changer de couleur, au gré de l'utilisateur, après illumination sous un rayonnement lumineux particulier.
La présente invention a donc pour but de proposer une composition cosmétique qui comprend de tels agents de coloration, et qui est donc susceptible de changer de couleur de manière rapide, en fonction de la nature et/ou de l'intensité d'un rayonnement lumineux particulier, mais au gré de l'utilisateur, tout en présentant de bonnes propriétés cosmétiques.

L'invention a donc pour objet une composition cosmétique comprenant, dans un milieu cosmétiquement acceptable, au moins un agent de coloration photochrome thermiquement irréversible, choisi parmi les diaryléthènes de formule (I) : 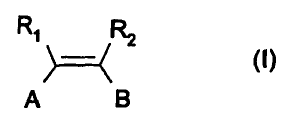 dans laquelle :
- les radicaux R1 et R2, toujours en relation "cis" par rapport à la double liaison, sont, indépendamment l'un de l'autre, choisis parmi les radicaux alkyles en C1-C16, éventuellement fluorés ou perfluorés, ou le radical nitrile CN; ou forment ensemble un cycle à 5 ou 6 atomes de carbone, éventuellement fluoré ou perfluoré;
   ou forment ensemble un cycle à 5 atomes de carbone d'anhydride, et notamment selon la formule suivante : 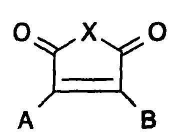 dans laquelle X peut être un atome d'oxygène ou un radical -NR, avec R représentant un radical alkyle et/ou hydroxyalkyle en C2-C16;
- les radicaux A et B, égaux ou différents, représentent un cycle à 5 atomes ou un bicycle à 5 et 6 atomes, selon les structures suivantes : 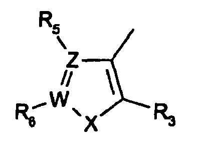 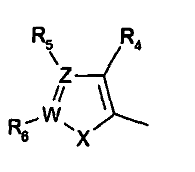 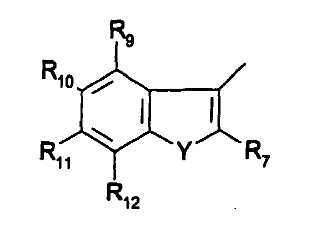 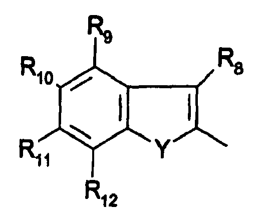 dans lesquelles :

- X et Y, égaux ou différents, représentent un atome d'oxygène, de soufre, d'une forme oxydée du soufre, ou de sélénium,
- Z et W, égaux ou différents, représentent un atome de carbone ou d'azote,
- les radicaux R₃ à R₁₂, égaux ou différents, représentent un hydrogène, un groupe alkyle ou alkoxy, linéaire ou ramifié, en C₁-C₁₆, un halogène, un groupe fluoré ou perfluoré linéaire ou ramifié en C₁-C₄, un groupe carboxylique, un groupe alkyle carboxylique en C₁-C₁₆, un groupe mono- ou dialkylamino en C₁-C₁₆, un groupe nitrile; éventuellement substitués par un groupe phényle, naphtalène ou un hétérocycle (pyridine, quinoléine, thiophène).
- les groupes A et B pouvant être séparés du cycle par une ou deux doubles liaisons,
   et/ou les fulgides de formule (II) : 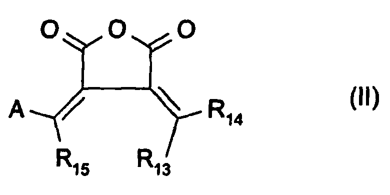
   dans laquelle :
   - le groupe A a la même signification que ci-dessus.
   - les groupes R₁₃ à R₁₅, égaux ou différents, représentent un groupe alkyle linéaire
      ou ramifié en C₁-C₁₆, ou bien les groupes R₁₃ et R₁₄ forment un cycle de 3 à 12 atomes de carbone tel qu'un cyclopropane ou un adamantylène.
Un autre objet de l'invention est l'utilisation d'un agent de coloration photochrome tel que défini ci-dessus comme agent de coloration dans une composition cosmétique.

Un autre objet de l'invention est un procédé de maquillage et/ou de coloration temporaire d'un support choisi parmi les muqueuses, les semi-muqueuses, la peau et/ou les phanères, dans lequel on applique sur ledit support une composition cosmétique telle que définie ci-dessus.

Dans la suite de la présente description, on entend par 'agent de coloration photochrome thermiquement irréversible' un composé qui possède des propriétés photochromes thermiquement irréversibles.
Le test pour déterminer si un composé photochrome est thermiquement réversible ou irréversible est le suivant : on soumet le composé à tester à une irradiation par un rayonnement UV pendant quelques minutes, de préférence pendant 1 minute, puis on détermine sa couleur à l'aide d'un colorimètre MINOLTA CM 2002; on exprime les valeurs en H, V, C dans la notation MUNSELL (selon la norme ASTM D 1535-68).
Ainsi, H désigne la nuance ou HUE, V désigne l'intensité ou VALUE, et C désigne la pureté ou CHROMATICITE.
On obtient ainsi une valeur A qui est correspond au maximum de la couleur que l'on peut obtenir.
On laisse ensuite ledit composé dans l'obscurité totale pendant 30 minutes à 25°C, puis on détermine à nouveau sa couleur selon la méthode ci-dessus; on obtient ainsi une valeur B.
Lorsque la valeur B est au moins égale à 50% de la valeur A, on considère que le composé est thermiquement irréversible.

Sont notamment exclus du cadre de la présente invention les composés photochromes thermiquement réversibles, tels que l'oxyde de titane dopé, les spiropyrannes, les spirooxazines ou les chromènes.

Sans être tenu par la présente explication, on peut illustrer la différence de comportement entre les agents de coloration photochromes selon l'invention et les composés photochromes selon l'art antérieur de la manière suivante.
Lorsqu'on l'applique sur un support, une composition cosmétique selon l'invention, on peut obtenir, selon le choix de l'agent de coloration photochrome, un film incolore ou très faiblement coloré. Ce film reste incolore ou faiblement coloré quelle que soit l'intensité lumineuse de l'environnement; lorsque l'utilisateur le souhaite, il peut modifier la couleur du film, par exemple obtenir un film de couleur rouge, en soumettant le support à un rayonnement UV particulier, de longueur d'onde donnée. Le film devient rouge et le reste aussi longtemps que souhaité; il est ensuite possible de faire à nouveau changer la couleur dudit film, en le faisant redevenir incolore ou légèrement coloré, par illumination sous un rayonnement de longueur d'onde donnée.

Il est clair que les couleurs susceptibles d'être conférées au film dépendent de la nature des agents de coloration; de même, les longueurs d'ondes nécessaires pour faire varier ces couleurs sont spécifiques à chaque agent de coloration.

Par opposition, une composition comprenant un composé photochrome selon l'art antérieur, par exemple un spiropyranne ou un oxyde de titane dopé, donnera après application un film incolore ou faiblement coloré; lorsque le support sera au soleil, le film deviendra, par exemple, de couleur rouge, pour revenir automatiquement et très rapidement incolore ou faiblement coloré lorsque le support sera à l'ombre ou dans un espace peu ou pas éclairé.

Ce phénomène de 'décoloration' à l'ombre est maîtrisé par l'utilisation d'agent de coloration photochrome selon l'invention.

La composition cosmétique selon l'invention trouve notamment une application particulièrement intéressante dans le domaine du maquillage de la peau, des semi-muqueuses, des muqueuses et/ou des phanères.
On entend notamment par muqueuse, la partie interne de la paupière inférieure; parmi les semi-muqueuses, on entend plus particulièrement les lèvres du visage.
On entend par phanères, les systèmes pileux constitués notamment par la chevelure humaine, les poils, les cils et les sourcils, ainsi que les ongles d'origine naturelle ou artificielle.

Les composés utilisés dans le cadre de la présente invention présentent notamment l'avantage d'être solubles dans les solvants organiques usuellement employés en cosmétique, tels que les alcools et/ou les esters.

La composition selon l'invention peut se présenter sous toute forme galénique cosmétiquement acceptable, telle que sous forme d'une lotion, suspension, dispersion, solution en milieu solvant ou hydroalcoolique, éventuellement multiphasée; sous forme d'un gel, d'une mousse, d'un spray, d'une émulsion huile-dans-eau, eau-dans-huile, ou multiple; sous forme de poudre libre, compacte ou coulée; sous forme d'un solide ou d'une pâte anhydre.
L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Parmi les agents de coloration photochromes utilisables dans le cadre de la présente invention, on retient les diaryléthènes de formule (I) et/ou les fulgides de formule (II).

Les diaryléthènes sont représentés par la formule (I) suivante : 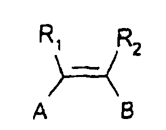 dans laquelle les radicaux R1 et R2 sont toujours en relation "cis" par rapport à la double liaison.

Ces radicaux R1 et R2 sont, indépendamment l'un de l'autre, choisis parmi les radicaux alkyles en C1-C16, éventuellement fluorés ou perfluorés, ou nitriles.
On peut notamment citer les composés de formule suivante :

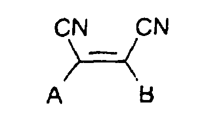

Ils peuvent également former un cycle à 5 ou 6 atomes de carbone, éventuellement fluoré ou perfluoré, notamment selon la formule suivante : 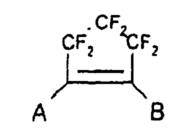 ou former un cycle à 5 atomes de carbone d'anhydride, et notamment selon la formule suivante : 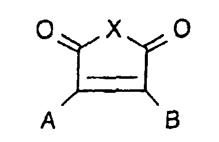 dans laquelle X peut être un atome d'oxygène ou un radical -NR, avec R représentant un radical alkyle et/ou hydroxyalkyle en C2-C16.

Les radicaux A et B peuvent être égaux ou différents et représentent un cycle à 5 atomes ou un bicycle à 5 et 6 atomes, selon les structures suivantes : 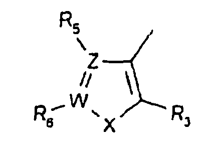 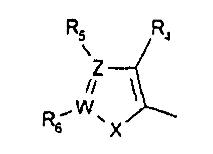 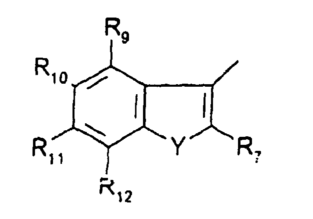 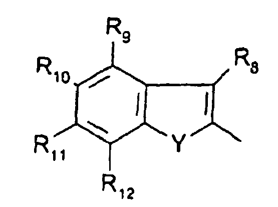 dans lesquelles :
- X et Y peuvent être égaux ou différents, et représentent un atome d'oxygène, de soufre, d'une forme oxydée du soufre, d'azote ou de sélénium,
- Z et W peuvent être égaux ou différents, et représentent un atome de carbone ou d'azote,
- les radicaux R₃ à R₁₂ peuvent être égaux ou différents, et représentent un hydrogène, un groupe alkyle ou alkoxy, linéaire ou ramifié, en C₁-C₁₆, un halogène, un groupe fluoré ou perfluoré linéaire ou ramifié en C₁-C₄, un groupe carboxylique, un groupe alkyle carboxylique en C1-C16, un groupe mono- ou dialkylamino en C1-C16, un groupe nitrile; un groupe phényle, naphtalène ou un hétérocycle (pyridine, quinoléïne, thiophène) peut être substitué sur ces radicaux.

Les groupes A et B peuvent être séparés du cycle par une ou deux doubles liaisons.

Il doit toujours y avoir sur les positions ortho de la jonction, entre la double liaison et les restes A et B, un groupe différent de l'hydrogène, par exemple CH3, CN ou CO2Et, c'est-à-dire que les groupes R3 ou R5, R4, R7 et R8 doivent être différents de la signification hydrogène.

On a constaté que les composés appartenant à la famille des diaryléthènes présentent des propriétés intéressantes, notamment qu'ils peuvent se transformer quantitativement en 10⁻¹² seconde et que leur forme cyclisée est thermiquement stable à 80° C pendant plus de trois mois.

A titre d'exemple, on peut citer le composé suivant qui peut passer de l'incolore au rouge de la manière suivante, après irradiation à 404-436 nm (retour à 546-578 nm) :

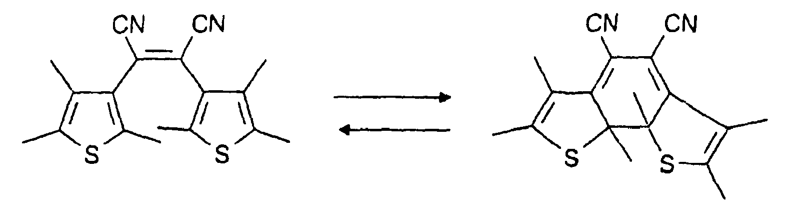

Les fulgides sont représentés par la formule suivante : 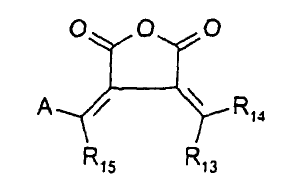 dans laquelle
. le groupe A a la même signification que ci-dessus.
. les groupes R₁₃ à R₁₅ peuvent être égaux ou différents, et représentent un groupe alkyle linéaire ou ramifié en C₁-C₁₆, ou bien les groupes R₁₃ et R₁₄ peuvent former un cycle de 3 à 12 atomes de carbone tel qu'un cyclopropane ou un adamantylène.

L'agent de coloration photochrome peut être présent dans la composition à une teneur de 0,05-30% en poids, de préférence 0,1-10% en poids. L'homme du métier pourra déterminer la teneur optimale en agent de coloration photochrome, de manière à obtenir d'une part une composition cosmétiquement acceptable, et d'autre part, l'effet de coloration souhaité.

La composition selon l'invention contient en outre un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.
Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; un spray; une poudre libre, compacte ou coulée; une pâte anhydre.

Ainsi, la composition selon l'invention peut comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa). De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane.
On peut également citer les huiles volatiles hydrocarbonées, telles que les isoparaffines et notamment l'isododécane; et des huiles fluorées comme celle commercialisée sous la dénomination GALDEN® (MONTEFLUOS).

On peut également utiliser des huiles non volatiles, parmi lesquelles on peut citer:
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule : 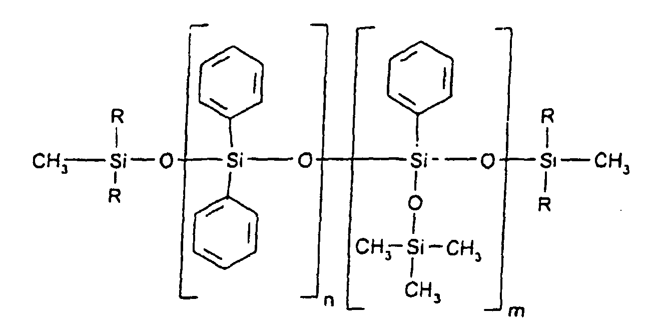 dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.
On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylene et les cires obtenues par synthèse de Fischer-Tropsch; des huiles hydrogénées concrètes à 25°C; des lanolines; des esters gras concrets à 25°C; les cires de silicone: les cires fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther.
Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.
Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.

La composition selon l'invention peut également comprendre une phase aqueuse, qui peut comprendre de l'eau, une eau florale telle que l'eau de bleuet et/ou une eau minérale telle que l'eau de VITTEL, l'eau de LUCAS ou l'eau de LA ROCHE POSAY.
Ladite phase aqueuse peut comprendre de 0 % à 14 % en poids, par rapport au poids total de la phase aqueuse, d'un monoalcool inférieur en C₂-C₆ et/ou d'un polyol tel que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition.
Parmi les tensioactifs anioniques qui peuvent être utilisés seuls ou en mélange, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, α-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates.
Le radical alcoyle ou acyle dans tous ces composés désigne généralement une chaîne de 12 à 18 atomes de carbone.
On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule :
Alk-(OCH₂-CH₂)ₙ-OCH₂-COOH sous forme acide ou salifiée où le substituant Alk correspond à une chaîne linéaire ayant de 12 à 18 atomes de carbone et où n est un nombre entier compris entre 5 et 15.
Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un α-diol, d'un monoalcool, d'un alcoylphénol, d'un amide ou d'un diglycolamide avec le glycidol ou un précurseur de glycidol.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids, par rapport au poids total de l'émulsion. L'agent épaississant peut être choisi parmi :
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.
L'agent épaississant peut être présent dans la phase grasse et/ou dans la phase aqueuse.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque.
Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène.
Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes.
On peut en particulier citer les copolymères d'acide (méth)acrylique et d'au moins un monomère ester d'acide (méth)acrylique linéaire, ramifié ou cyclique et/ou d'au moins un monomère amide d'acide (méth)acrylique mono ou di-substitué linéaire, ramifié ou cyclique; les copolymères acide (méth)acrylique/(meth)acrylate de tertio-butyle et/ou (méth)acrylate d'isobutyle/(méth)acrylate d'alkyle en C₁-C₄; les terpolymères et tétrapolymères acide (meth)acrylique/acrylate d'éthyle/ méthacrylate de méthyle; les tétrapolymères méthacrylate de méthyle/acrylate de butyle ou d'éthyle/acrylate ou méthacrylate d'hydroxyéthyle ou de 2-hydroxypropyle/acide (meth)acrylique; les copolymères d'acide acrylique et de méthacrylate d'alkyle en C₁-C₄ ; les terpolymères de vinyl pyrrolidone, d'acide acrylique et de méthacrylate d'alkyle en C₁₋₂₀, les copolymères amphotères; les esters vinyliques d'acide ramifiés; les esters vinyliques d'acide benzoïque; les copolymères d'acide (meth)acrylique et d'au moins un monomère oléfinique; les copolymères de monoacide vinylique et/ou de monoacide allylique.
Parmi les résines, on peut citer les résines du type aryl-sulfonamide formaldéhyde ou aryl-sulfonamide époxy; les résines du type acrylique, styrénique, acrylate-styrénique et vinylique.
La composition peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triétyl-2 hexyle, le camphre; les éthers de glycol; l'huile de ricin oxyéthylénée à 40 moles d'oxyde d'éthylène; le propylène glycol; le butyl glycol ; l'éthylène glycol monométhyléther acétate; les éthers de propylène glycol; les éthers esters de propylène glycol et d'éthylène glycol; les esters de diacides tels que les phtalates et adipates de diéthyle, dibutyle et de diisopropyle, les tartrates de diéthyle et dibutyle, les succinates de diéthyle et de dibutyle, les sébacates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40 % en poids par rapport au poids total de la composition.
Lorsque les compositions se présentent sous forme d'un vernis à ongles anhydre, le système solvant peut représenter environ de 55 % à 90 % en poids par rapport au poids total du vernis. Ce système solvant peut être constitué par un mélange de divers solvants organiques volatils tels que les cétones notamment l'acétone, la méthyléthylcétone, la méthylisobutylcétone; les acétates notamment l'acétate d'éthyle, l'acétate de butyle, l'acétate de méthoxy-2 éthyle, l'acétate de méthyle, l'acétate d'amyle et l'acétate d'isopropyle. Le système solvant peut également comprendre un diluant tel que l'hexane ou l'octane ou encore un hydrocarbure aromatique tel que le toluène ou le xylène en une proportion notamment de 10 à 35 % en poids par rapport au poids total du vernis.

La composition peut comprendre en outre une phase particulaire, qui peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. Ils peuvent se présenter sous forme de poudre ou de pâte pigmentaire.
On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques, etc.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.
Selon le type de formulation, la phase pulvérulente peut représenter de 0,01 à 99% en poids de la composition.

La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des colorants, des acides gras essentiels, des sphingolipides, des agents autobronzants tels que la DHA, des filtres solaires, des agents antimousses, des agents séquestrants, des antioxydants.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques selon l'invention sont essentiellement celles concernant le maquillage du visage, c'est-à-dire les fards à paupières, eye-liners, mascaras, poudres, fonds de teint, fards à joues, "Blush", crèmes teintées, rouges à lèvres, sticks à lèvres ou sticks anti-cernes, mais également le maquillage des cheveux notamment des gels, crèmes ou mousses pour la coloration temporaire des cheveux, et le maquillage des ongles notamment les vernis à ongles anhydres.

Les compositions anhydres peuvent se présenter sous forme de poudre libre ou compactée, de fard solide, pâteux ou liquide pouvant contenir un liant qui peut représenter de préférence de 0,01 à 95 % en poids par rapport au poids total de la composition. Les compositions capillaires selon l'invention peuvent se présenter sous forme de compositions pressurisée en aérosol en présence d'un agent propulseur. Les compositions selon l'invention peuvent également se présenter sous forme d'un gel, d'une solution aqueuse ou hydroalcoolique d'un ou plusieurs polymères hydrosolubles tels que les dérivés de l'acide polyacrylique ou sous forme de gels émulsionnés obtenus par dispersion d'huiles dans des gels à l'aide d'émulsionnants tels que les Pemulens de la Société GOODRICH. Les compositions selon l'invention peuvent encore se présenter sous forme de stick et peuvent contenir des charges et un liant qui peuvent représenter de préférence de 0,01 à 95 % en poids par rapport au poids total de la composition.

L'invention est illustrée plus en détail dans les exemples suivants.

### Exemple 1

On prépare une crème pour le visage sous forme d'une émulsion huile dans eau, ayant la composition suivante (% en poids):
- Huiles (minérale, de silicone et hydrocarbonées) 20%
- Magnésium aluminium silicate gel à 5 % 20%
- Gomme de cellulose 3,5%
- Tensioactif 4%
- Conservateurs q.s.
- Eau qsp 100%
- Agent de coloration selon l'invention 2%
2,3-bis (2,4,5-triméthyl-thiophen-3-yl)-but-2-ènedinitrile

Lorsque l'on applique la composition selon l'invention sur le dos de la main, et après irradiation, on obtient un effet bonne mine.

### Exemple 2

On prépare une lotion capillaire ayant la composition suivante :
- Polymère filmogène à 30% dans une solution hydroalcoolique 50/50 9%
- Ethanol 35%
- Eau qsp 100%
- Agent de coloration selon l'invention 5%
2,3-bis (2,4,5-triméthyl-thiophen-3-yl)-but-2-ènedinitrile

Appliquée sur des cheveux châtains, sans rinçage, cette lotion confère à la chevelure après séchage et irradiation, des reflets.

### Exemple 3

On prépare un vernis à ongles anhydre ayant la composition suivante :
- Nitrocellulose 15%
- Résine toluène sulfonamide formaldéhyde 10%
- Acétylcitrate de tributyle 6%
- Acétate de butyle 20%
- Acétate d'éthyle 10%
- Toluène qsp 100%
- Agent de coloration selon l'invention 4%
2,3-bis (2,4,5-triméthyl-thiophen-3-yl)-but-2-ènedinitrile

Le vernis obtenu est incolore à légèrement jaune dans le flacon.
En appliquant directement ce vernis à ongles sur l'ongle, et après irradiation, on obtient un léger effet rose.

Ce vernis peut également être appliqué sur de faux ongles.
On peut utiliser des caches prédécoupés à appliquer sur les ongles avant illumination, de manière à permettre de dessiner des motifs ou d'écrire sur l'ongle.
On peut également utiliser des agents de coloration différents pour chaque ongle, de manière à obtenir des ongles de couleur différente.

### Exemple 4

On prépare un vernis à ongles aqueux ayant la composition suivante:
- dispersion aqueuse de polyester-polyuréthanne (35,8% de matière sèche) 55%
- dispersion aqueuse de polymère acrylique (matière sèche: 45,5%) 18%
- épaississant associatif polyuréthanne 0,8%
- eau qsp 100%
- agent de coloration selon l'invention 3%
2,3-bis (2,4,5-triméthyl-thiophen-3-yl)-but-2-ènedinitrile

Ce vernis peut également être appliqué sur de faux ongles.

### Exemple 5

On prépare des compositions de vernis à ongles comprenant les ingrédients suivants :
- nitrocellulose 15%
- résine toluène sulfonamide formaldéhyde 10%
- acétylcitrate de tributyle 6%
- acétate de butyle 20%
- acétate d'éthyle 10%
- 2,3-bis (2,4,5-triméthyl-thiophen-3-yl)-but-2-ènedinitrile x%
- toluène qsp 100%

Les compositions obtenues sont de couleur légèrement jaune à jaune dans le flacon conservé à l'abri de la lumière, et de couleur rouge si le flacon est exposé à la lumière du jour.
En appliquant ces compositions jaune sur l'ongle, et après irradiation par une source lumineuse ayant une longueur d'ondes d'environ 400 nm, on obtient un effet rose à rouge.
Ces compositions peuvent également être appliquées sur de faux ongles.

Les compositions préparées ci-dessus sont appliquées sur de faux ongles et irradiées à l'aide des deux lampes suivantes :
- lampe PHILIPS SOX-E18, de longueur d'onde 596 nm
- lampe UV avec filtre pour chromatographie couche mince, de longueur d'onde 365 nm

On mesure ensuite leur couleur à l'aide d'un colorimètre MINOLTA CM 2002; on exprime les valeurs en H, V, C dans la notation MUNSELL (selon la norme ASTM D 1535-68).

On obtient les résultats suivants :
1- après irradiation à 596 nm

| x(%) | H | V | C | L | a | b |
|---|---|---|---|---|---|---|
| 0 | 4,73 | 8,0 | 3,0 | 80,40 | -2,01 | 20,77 |
| 0,125 | 6,73 | 7,4 | 3,2 | 75,56 | -4,09 | 23,03 |
| 0,25 | 7,77 | 7,3 | 3,6 | 73,94 | -5,67 | 26,08 |
| 0,5 | 6,87 | 7,2 | 5,0 | 72,88 | -5,88 | 35,55 |
| 1,125 | 6,27 | 6,7 | 5,9 | 67,82 | -5,03 | 42,07 |
| 2,5 | 6,67 | 7,9 | 8,3 | 79,90 | -7,97 | 59,10 |
| 5 | 6,17 | 7,4 | 8,8 | 74,97 | -6,44 | 62,34 |
| 10 | 6,90 | 7,2 | 8,1 | 72,68 | -7,81 | 57,97 |

2- après irradiation à 365 nm

| x (%) | H | V | C | L | a | b |
|---|---|---|---|---|---|---|
| 0 | 4,80 | 7,9 | 2,9 | 80,36 | -2,03 | 20,94 |
| 0,125 | 3,17 | 5,9 | 6,2 | 59,83 | 19,85 | 29,89 |
| 0,25 | 4,77 | 4,7 | 9,4 | 47,32 | 39,68 | 19,76 |
| 0,5 | 8,90 | 4,6 | 8,9 | 47,05 | 35,23 | 29,73 |
| 1,125 | 7,83 | 4,3 | 8,8 | 44,40 | 36,42 | 26,70 |
| 2,5 | 9,67 | 5,0 | 10,4 | 51,34 | 39,78 | 37,93 |
| 5 | 9,43 | 4,8 | 10,4 | 48,49 | 39,85 | 36,86 |
| 10 | 0,00 | 4,7 | 9,5 | 48,11 | 35,76 | 35,56 |

3- après irradiation à la lumière du jour

| x(%) | H | V | C | L | a | b |
|---|---|---|---|---|---|---|
| 0 | 4,73 | 8,0 | 3,0 | 80,40 | -2,01 | 20,77 |
| 0,125 | 0,23 | 6,8 | 4,6 | 69,35 | 4,07 | 30,28 |
| 0,25 | 2,13 | 5,9 | 5,4 | 60,22 | 18,56 | 24,31 |
| 0,5 | 0,07 | 5,0 | 7,9 | 51,24 | 29,95 | 30,01 |
| 1,125 | 5,40 | 3,9 | 10,0 | 39,32 | 42,83 | 22,31 |
| 2,5 | 5,70 | 4,1 | 12,1 | 42,52 | 51,28 | 29,01 |
| 5 | 4,60 | 3,5 | 11,7 | 36,00 | 50,09 | 23,20 |
| 10 | 4,50 | 3,5 | 11,0 | 35,81 | 47,52 | 21,51 |

## Revendications

1. Composition cosmétique comprenant, dans un milieu cosmétiquement acceptable, au moins un agent de coloration photochrome thermiquement irréversible choisi parmi les diaryléthènes de formule (1): 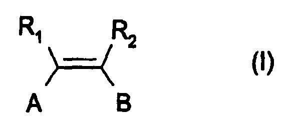 dans laquelle :
- les radicaux R1 et R2, toujours en relation "cis" par rapport à la double liaison, sont, indépendamment l'un de l'autre, choisis parmi les radicaux alkyles en C1-C16, éventuellement fluorés ou perfluorés, ou le radical nitrile CN; ou forment ensemble un cycle à 5 ou 6 atomes de carbone, éventuellement fluoré ou perfluoré; ou forment ensemble un cycle à 5 atomes de carbone d'anhydride, et notamment selon la formule suivante : 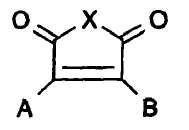
dans laquelle X peut être un atome d'oxygène ou un radical -NR, avec R représentant un radical alkyle et/ou hydroxyalkyle en C2-C16;
- les radicaux A et B, égaux ou différents, représentent un cycle à 5 atomes ou un bicycle à 5 et 6 atomes, selon les structures suivantes : 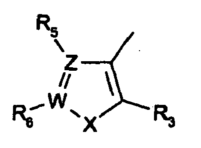 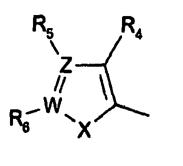 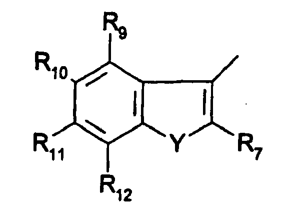 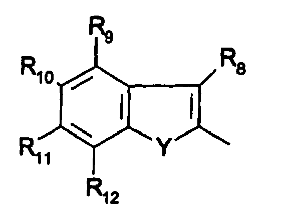
dans lesquelles :
- X et Y, égaux ou différents, représentent un atome d'oxygène, de soufre, d'une forme oxydée du soufre, ou de sélénium,
- Z et W, égaux ou différents, représentent un atome de carbone ou d'azote,
- les radicaux R₃ à R₁₂, égaux ou différents, représentent un hydrogène, un groupe alkyle ou alkoxy, linéaire ou ramifié, en C₁-C₁₆, un halogène, un groupe fluoré ou perfluoré linéaire ou ramifié en C₁-C₄, un groupe carboxylique, un groupe alkyle carboxylique en C₁-C₁₆, un groupe mono- ou dialkylamino en C₁-C₁₆, un groupe nitrile; éventuellement substitués par un groupe phényle, naphtalène ou un hétérocycle (pyridine, quinoléine, thiophène).
- les groupes A et B pouvant être séparés du cycle par une ou deux doubles liaisons,
et/ou les fulgides de formule (II) : 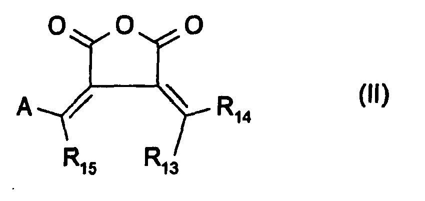
dans laquelle :
- le groupe A a la même signification que ci-dessus.
- les groupes R₁₃ à R₁₅, égaux ou différents, représentent un groupe alkyle linéaire ou ramifié en C₁-C₁₆, ou bien les groupes R₁₃ et R₁₄ forment un cycle de 3 à 12 atomes de carbone tel qu'un cyclopropane ou un adamantylène.

2. Composition selon la revendication 1, dans laquelle l'agent de coloration photochrome est de formule: 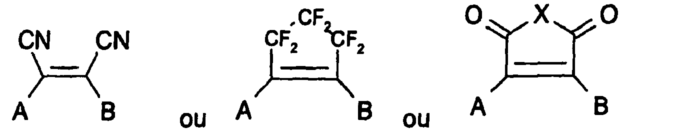 dans laquelle :
- X peut être un atome d'oxygène ou un radical -NR₃, avec R représentant un radical alkyle et/ou hydroxyalkyle en C2-C16;
- A et B étant tels que définis en revendication 1.

3. Composition selon l'une des revendications précédentes, dans laquelle le composé photochrome est de formule :

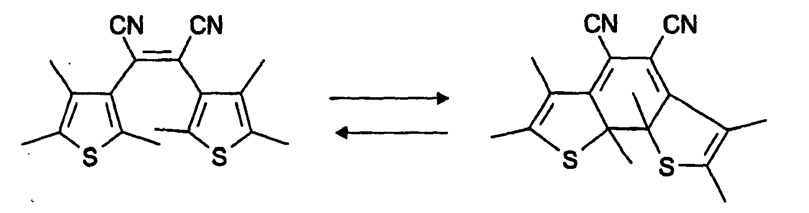

4. Composition selon l'une des revendications précédentes, dans laquelle l'agent de coloration photochrome est présent à une teneur de 0,05-30% en poids, de préférence 0,1-10% en poids.

5. Composition selon l'une des revendications précédentes, comprenant en outre une phase grasse comprenant des corps gras liquides à 25°C, tels que des huiles d'origine animale, végétale, minérale ou synthétique; des corps gras solides à 25°C tels que des cires d'origine animale, végétale, minérale ou synthétique; des corps gras pâteux; des gommes; des huiles volatiles; et leurs mélanges.

6. Composition selon l'une des revendications précédentes, comprenant en outre une phase aqueuse et/ ou un tensioactif et/ou un épaississant et/ou un polymère filmogène et/ou une phase particulaire qui comprend des pigments et/ou des nacres et/ou des charges

7. Composition selon l'une des revendications précédentes, se présentant sous la forme d'une lotion, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; un spray; une poudre libre, compacte ou coulée; une pâte anhydre.

8. Composition selon l'une des revendications précédentes, se présentant sous la forme d'une composition de maquillage du visage, telle que fard à paupières, eye-liner, mascara, poudre, fond de teint, fard à joues, "Blush", crème teintée, rouge à lèvres, stick à lèvres ou stick anti-cemes; d'une composition de maquillage des cheveux telle que gel, crème ou mousse pour la coloration temporaire des cheveux; d'une composition de maquillage des ongles telle que vernis à ongles.

9. Utilisation d'un agent de coloration photochrome tel que défini dans l'une des revendications 1 à 3, comme agent de coloration dans une composition cosmétique.

10. Procédé de maquillage et/ou de coloration temporaire d'un support choisi parmi les muqueuses, les semi-muqueuses, la peau et/ou les phanères, dans lequel on applique sur ledit support une composition cosmétique telle que définie dans l'une des revendications 1 à 8.

## Claims

1. Cosmetic composition comprising, in a cosmetically acceptable medium, at least one thermally irreversible photochromic colouring agent chosen from the diarylethenes of formula (I): 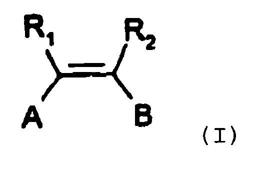 in which:
- the radicals R₁ and R₂, which are always in a "cis" relationship relative to the double bond, are, independently of each other, chosen from optionally fluorinated or perfluorinated C₁-C₁₆ alkyl radicals, and the nitrile radical CN; or together form an optionally fluorinated or perfluorinated ring containing 5 or 6 carbon atoms; or together form an anhydride ring containing 5 carbon atoms, and in particular according to the following formula: 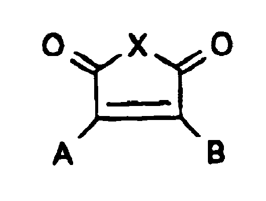
in which X can be an oxygen atom or a radical -NR, with R representing a C₂-C₁₆ alkyl and/or hydroxyalkyl radical,
- the radicals A and B, which may be identical or different, represent a ring containing 5 atoms or a bicycle containing 5 and 6 atoms, according to the following structures: 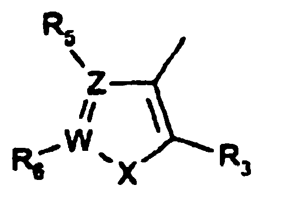 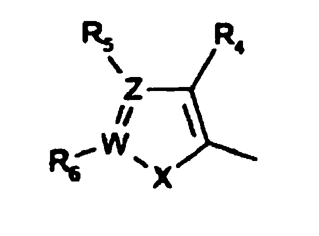 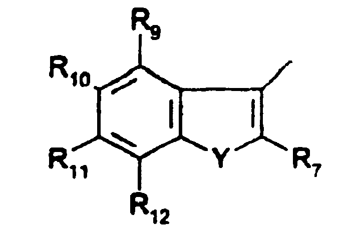 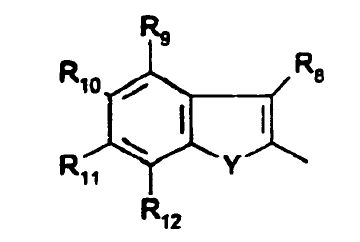
in which:
- X and Y, which may be identical or different, represent an oxygen or sulphur atom or an oxidized form of sulphur, or of selenium,
- Z and W, which may be identical or different, represent a carbon or nitrogen atom,
- the radicals R₃ to R₁₂, which may be identical or different, represent a hydrogen, a linear or branched C₁-C₁₆ alkyl or alkoxy group, a halogen, a linear or branched C₁-C₄ fluoro or perfluoro group, a carboxylic group, a C₁-C₁₆ alkylcarboxylic group, a C₁-C₁₆ mono- or dialkylamino group, a nitrile group; optionally substituted by a phenyl or naphthalene group or a heterocycle (pyridine, quinoline, thiophene),
- it being possible for the groups A and B to be separated from the ring by one or two double bonds,
and/or the fulgides of formula (II): 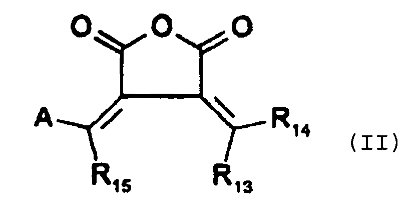
in which
. the group A has the same meaning as above,
. the groups R₁₃ to R₁₅, which may be identical or different, represent a linear or branched C₁-C₁₆ alkyl group, or alternatively the groups R₁₃ and R₁₄ form a ring of 3 to 12 carbon atoms, such as a cyclopropane or an adamantylene.

2. Composition according to Claim 1, in which the photochromic colouring agent has the formula: 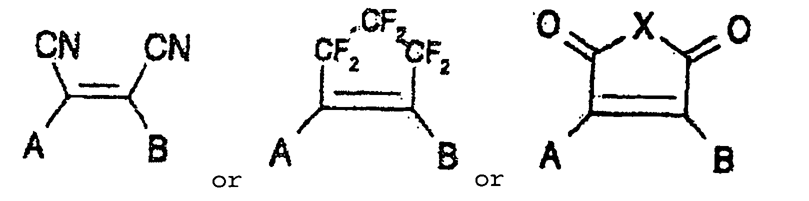 in which:
- X can be an oxygen atom or a radical -NR₃, with R representing a C₂-C₁₆ alkyl and/or hydroxyalkyl radical;
- A and B being as defined in Claim 1.

3. Composition according to either of the preceding Claims, in which the photochromic compound has the formula:

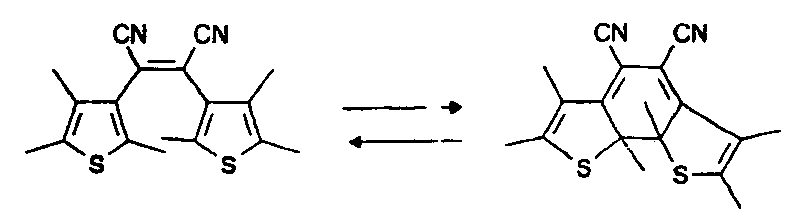

4. Composition according to one of the preceding claims, in which the photochromic colouring agent is present at a content of 0.05-30% by weight, preferably 0.1-10% by weight.

5. Composition according to one of the preceding claims, also comprising a fatty phase comprising fatty substances which are liquid at 25°C, such as oils of animal, plant, mineral or synthetic origin; fatty substances which are solid at 25°C, such as waxes of animal, plant, mineral or synthetic origin; pasty fatty substances; gums; volatile oils; and mixtures thereof.

6. Composition according to one of the preceding claims, also comprising an aqueous phase and/or a surfactant and/or a thickener and/or a film-forming polymer and/or a particulate phase which comprises pigments and/or nacres and/or fillers.

7. Composition according to one of the preceding claims, which is in the form of a lotion, a suspension, a dispersion or a solution in aqueous-alcoholic or solvent medium, which may be thickened or even gelled; an oil-in-water, water-in-oil or multiple emulsion; a gel or a mousse; an emulsified gel; a spray; a free, compact or cast powder; an anhydrous paste.

8. Composition according to one of the preceding claims, which is in the form of a make-up composition for the face, such as an eyeshadow, eyeliner, mascara, powder, foundation, blusher, tinted cream, lipstick, lip pencil or concealer stick; a make-up composition for the hair, such as a gel, cream or mousse for temporarily colouring the hair; a make-up composition for the nails, such as a nail varnish.

9. Use of a photochromic colouring agent as defined in one of Claims 1 to 3, as a colouring agent in a cosmetic composition.

10. Process for temporarily colouring and/or making up a support chosen from mucous membranes, semi-mucous membranes, the skin and/or the integuments, in which a cosmetic composition as defined in one of Claims 1 to 8 is applied to the said support.

## Patentansprüche

1. Kosmetische Zusammensetzung, die in einem kosmetisch akzeptablen Medium mindestens einen thermisch irreversiblen photochromen Farbstoff enthält, der ausgewählt wird unter
den Diarylethenen der folgenden Formel (I) 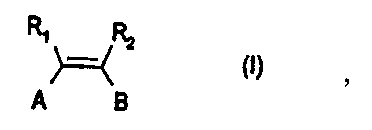 in der bedeuten:
- die Reste R₁ und R₂, immer in "cis"-Anordnung, bezogen auf die Doppelbindung, unabhängig voneinander Reste, die unter den (C₁-C₁₆)-Alkylresten, die gegebenenfalls fluoriert oder perfluoriert sind, und dem Nitrilrest CN ausgewählt werden; oder Reste, die zusammen einen Ring mit 5 oder 6 Kohlenstoffatomen bilden, der gegebenenfalls fluoriert oder perfluoriert ist; oder Reste, die zusammen einen Ring eines Anhydrids mit 5 Kohlenstoffatomen bilden, insbesondere gemäß der folgenden Formel: 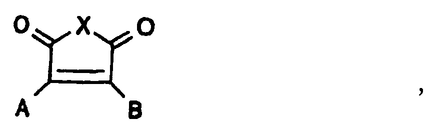 in der X ein Sauerstoffatom oder ein Rest -NR sein kann, worin R einen Alkylrest und/oder Hydroxyalkylrest bedeutet, der 2 bis 16 Kohlenstoffatome aufweist,
- die Reste A und B, gleich oder verschieden, einen Ring mit 5 Atomen oder ein bicyclisches Ringsystem mit 5 und 6 Atomen, gemäß den folgenden Strukturen 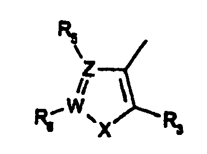 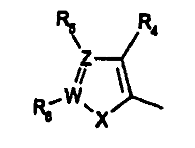 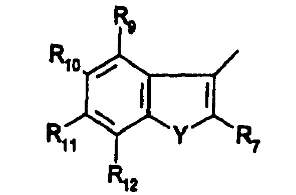 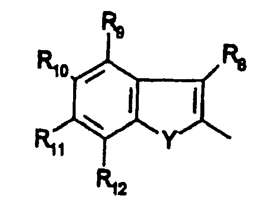 in denen bedeuten:
- X und Y, gleich oder verschieden, ein Sauerstoffatom, ein Schwefelatom, eine oxidierte Form des Schwefelatoms oder ein Selenatom,
- Z und W, gleich oder verschieden, ein Kohlenstoffatom oder ein Stickstoffatom,
- die Reste R₃ bis R₁₂, gleich oder verschieden, Wasserstoff, eine geradkettige oder verzweigte (C₁-C₁₆)-Alkyl- oder (C₁-C₁₆)-Alkoxygruppe, ein Halogenatom, eine geradkettige oder verzweigte, fluorierte oder perfluorierte (C₁-C₄)-Gruppe, eine Carboxygruppe, eine (C₁-C₁₆)-Carboxy-alkylgruppe, eine (C₁-C₁₆)-Mono- oder Dialkylaminogruppe, eine Nitrilgruppe; die gegebenenfalls mit einer Phenylgruppe, einer Naphthalingruppe oder einem Heterocyclus (Pyridin, Chinolin, Thiophen) substituiert sind,
- wobei die Gruppen A und B durch eine oder zwei Doppelbindungen vom Ring getrennt sein können.
und/oder den Fulgiden der Formel (II) 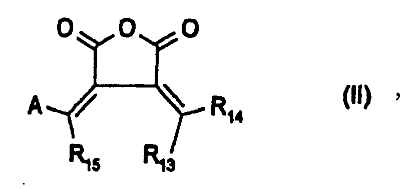 in der
- A die gleiche Bedeutung wie weiter oben hat,
- die Gruppen R₁₃ bis R₁₅, gleich oder verschieden, eine geradkettige oder verzweigte (C₁-C₁₆-)-Alkylgruppe bedeuten oder die Gruppen R₁₃ und R₁₄ einen Ring aus 3 bis 12 Kohlenstoffatomen bilden, wie ein Cyclopropan oder ein Adamantylen.

2. Zusammensetzung nach Anspruch 1, in der der photochrome Farbstoff die Formel 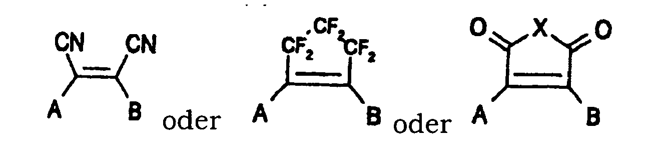 hat, in der
- X ein Sauerstoffatom oder ein Rest -NR₃ sein kann, wobei R einen Alkylrest und/oder Hydroxyalkylrest darstellt, der 2 bis 16 Kohlenstoffatomen aufweist;
- A und B wie in Anspruch 1 definiert sind.

3. Zusammensetzung nach einem der vorhergehenden Ansprüche, in der die photochrome Verbindung der Formel 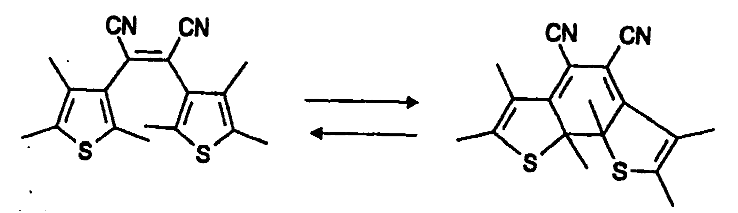 entspricht.

4. Zusammensetzung nach einem der vorhergehenden Ansprüche, wobei der photochrome Farbstoff in einem Gehalt von 0,05 bis 30 Gew.-% und vorzugsweise 0,1 bis 10 Gew.-% enthalten ist.

5. Zusammensetzung nach einem der vorhergehenden Ansprüche, die außerdem eine Fettphase enthält, die enthält: Fettsubstanzen, die bei 25 °C flüssig sind, wie Öle tierischer, pflanzlicher, mineralischer oder synthetischer Herkunft; Fettsubstanzen, die bei 25 °C fest sind, wie Wachse tierischer, pflanzlicher, mineralischer oder synthetischer Herkunft; pastöse Fettsubstanzen; Gummen/Gummis; flüchtige Öle; und deren Gemische.

6. Zusammensetzung nach einem der vorhergehenden Ansprüche, die außerdem enthält: eine wäßrige Phase und/ oder einen grenzflächenaktiven Stoff und/oder ein Verdickungsmittel und/oder ein filmbildendes Polymer und/oder eine Partikelphase, die Pigmente und/oder Perlglanzpigmente und/oder Füllstoffe enthält.

7. Zusammensetzung nach einem der vorhergehenden Ansprüche, die in Form einer Lotion, einer Suspension, einer Dispersion, einer Lösung in einem Lösemittelmedium oder einem wäßrig-alkoholischen Medium, die gegebenenfalls verdickt oder sogar geliert ist; einer Ölin-Wasser-Emulsion, Wasser-in-Öl-Emulsion oder einer multiplen Emulsion; eines Gels oder eines Schaumes; eines emulgierten Gels, eines Sprays, eines losen, gepressten oder gegossenen Puders; einer wasserfreien Paste vorliegt.

8. Zusammensetzung nach einem der vorhergehenden Ansprüche, die in der Form einer Zusammensetzung zum Schminken des Gesichts, wie eines Lidschattens, eines Eyeliners, eines Mascaras, eines Puders, eines Make-ups, eines Rouge, eines "Blush", einer getönten Creme, eines Lippenstifts, Lippenkonturstifts oder eines Stifts gegen Augenringe; einer Zusammensetzung zum Schminken der Haare, wie eines Gels, einer Creme oder eines Schaumes für die temporäre Färbung der Haare; einer Zusammensetzung zum Schminken der Nägel, wie eines Nagellacks, vorliegt.

9. Verwendung eines wie in einem der Ansprüche 1 bis 3 definierten photochromen Farbstoffs als Farbstoff in einer kosmetischen Zusammensetzung.

10. Verfahren zum Schminken und/ oder temporären Färben eines Trägers, der unter den Schleimhäuten, den Halbschleimhäuten, der Haut und/ oder den Hautanhangsgebilden ausgewählt ist, bei dem eine wie in einem der Ansprüche 1 bis 8 definierte kosmetische Zusammensetzung auf den Träger aufgetragen wird.
